# EUROPÄISCHE PATENTSCHRIFT

(11) **EP 3 332 746 B1**
(45) Veröffentlichungstag und Bekanntmachung des Hinweises auf die Patenterteilung: **04.12.2019**
(21) Anmeldenummer: 16202345.1
(22) Anmeldetag: 06.12.2016
(51) Int. Cl.: A61F 2/36, A61F 2/30, A61F 2/46

(54) **REVISIONSPROTHESENSCHAFT FÜR EINE REVISIONS-GELENKENDOPROTHESE**
REVISION PROSTHESIS SHAFT FOR A REVISION JOINT ENDOPROSTHESIS
TIGE DE PROTHÈSE DE RÉVISION POUR UNE ENDOPROTHÈSE ARTICULAIRE DE RÉVISION

(43) Veröffentlichungstag der Anmeldung: 13.06.2018
(73) Patentinhaber: WALDEMAR LINK GmbH & Co. KG, 22339 Hamburg (DE)
(72) Erfinder: Link, Helmut D., 22397 Hamburg (DE)
(74) Vertreter: Glawe, Delfs, Moll

(56) Entgegenhaltungen:
- EP-A1- 0 319 171
- EP-A1- 0 359 485
- EP-A1- 2 491 893
- DE-A1- 4 320 086
- DE-U1- 29 907 259
- FR-A1- 2 674 744
- FR-A1- 2 875 397

## Beschreibung

Die Erfindung betrifft einen Revisionsprothesenschaft für eine Revisions-Gelenkendoprothese zur Verankerung in einem epimetaphysären Bereich eines langgestreckten Knochens. Der Revisionsprothesenschaft weist einen Halsteil am oberen Ende zur Aufnahme eines Gelenkstücks und einen sich an den Halsteil anschließenden Schaftteil auf, dessen Oberfläche zur adhäsionsmittel-freien (zementfreien) Befestigung in der Epimetaphyse ausgebildet ist. Die Länge des Schaftteils ist so bemessen, dass er mindestens in die Diaphyse des Knochens reicht.

Ein bei dem Ersatz (Revision) von Gelenkendoprothesen auftretendes Problem ist, dass der die Prothese aufnehmende Knochen häufig geschädigt ist. Grund hierfür sind insbesondere Defekte an der Knochensubstanz oder -oberfläche aufgrund der Extraktion der vorhandenen Prothese, ggf. noch verschlimmert durch weitere Defekte an dem Knochen. Um dennoch auch bei der Ersatz-Gelenkendoprothese eine ausreichend sichere Verankerung zu erreichen, sind die Schäfte der Revisionsprothesen besonders lang ausgeführt (Langschaft). Er ist so bemessen, dass er in einen tiefer gelegenen Bereich mit tragfähiger Knochensubstanz (Diaphyse) ragt. Bei der Diaphyse handelt es sich um den engen, meist zylindrischen Zentralbereich eines langgestreckten röhrenartigen Knochens, wie beispielsweise des Femurs.

Revisionsprothesensysteme mit einem solchen Revisionsschaft sind u. a. bekannt als das MP-Rekonstruktionsprothesensystem der Fa. Waldemar Link GmbH & Co KG, Hamburg (Deutschland). Hierbei ist der Schaft so ausgeführt, dass er in seinem oberen (proximalen) Bereich zementfrei implantiert werden kann. Dies ermöglicht dem geschädigten Knochen eine Regeneration. Die Länge des Schafts ist so groß bemessen, dass er in die

Diaphyse des Femurs reicht. Dort ist eine Befestigungsstrecke von 40 bis 80 mm erforderlich, um eine ausreichend sichere Verankerung zu erreichen.

Es hat sich in der klinischen Praxis gezeigt, dass bei Revisionsoperationen diese benötigte Befestigungsstrecke nicht immer gewährleistet werden kann. Dann kann eine ausreichend stabile Verankerung des Revisionsschafts nicht mehr erreicht werden. Dies kann in der Folge zur Lockerung des Schafts führen und sorgt für eine verringerte Lebenserwartung der Revisionsprothese. Das ist ein erheblicher Nachteil.

EP-A-0319171 offenbart eine Revisionsprothese mit einem Schaft der bis in die Diaphyse des Oberschenkelknochens reicht, und der mittels Knochenzement befestigt wird. FR-A-2674744 offenbart einen Prothesenschaft, der in seinem distalen Bereich mittels Knochenzement befestigt wird, und dessen proximaler Bereich zementfrei fixiert ist.

Der Erfindung liegt daher die Aufgabe zugrunde, einen verbesserten Revisionsschaft bereitzustellen, der diesen Nachteil vermeidet.

Die erfindungsgemäße Lösung liegt in den Merkmalen der unabhängigen Ansprüche. Vorteilhafte Weiterbildungen sind Gegenstand der abhängigen Ansprüche.

Bei einem Revisionsprothesenschaft einer Revisions-Gelenkendoprothese zur Verankerung in einem epimetaphysären Bereich eines langgestreckten Knochens, insbesondere Femur, der eine Epimetaphyse, eine Diaphyse und eine distale Epimetaphyse umfasst, wobei der Revisionsprothesenschaft einen Halsteil am oberen Ende zur Aufnahme eines Gelenkstücks und ein sich an den Halsteil anschließenden Schaftteil aufweist, dessen Oberfläche zur adhäsionsmittel-freien (zementfreien) Befestigung in der Epimetaphyse und Diaphyse ausgebildet ist und dessen Länge so bemessen ist, um in die Diaphyse des Knochens zu reichen, ist erfindungsgemäß vorgesehen, dass an dem fernen Ende des Schaftteils ein distal-epimetaphysärer Fortsatz für den Schaft vorgesehen ist, dessen Länge so bemessen ist, dass seine Spitze in die distale Epimetaphyse des Knochens reicht, wobei der Fortsatz zur Befestigung mit einem Adhäsionsmittel, insbesondere Knochenzement, in der distalen Epimetaphyse ausgebildet ist.

Nachfolgend seien zuerst einige verwendete Begriffe erläutert.

Unter einem Revisionsschaft wird ein Schaft einer Gelenkendoprothese verstanden, der zum Ersatz eines Schafts einer vorhandenen Gelenkendoprothese dient. Er ist dazu ausgebildet, auch in einem - durch Extraktion der vorigen Prothese und/oder weitere Defekte - geschädigten Knochen verankert zu werden.

Unter Halsteil wird ein Bereich der Gelenkendoprothese verstanden, an dem ein Gelenkelement angeordnet wird. Beispielsweise handelt es sich im Fall einer Femurkomponente einer Hüftprothese bei dem Halsteil um denjenigen oberen Bereich, auf den ein Kugelkopf als Gelenkelement aufgesteckt wird. Entsprechend wird unter Schaftteil der Schaftbereich verstanden, der bei einer Femurkomponente in den Markraum des Femurs eingeschoben wird.

Unter Adhäsionsmittel wird vorliegend ein durch Adhäsionswirkung bindendes Material verstanden, wie Knochenzement (z. B. aus Polymethylmethacrylate).

Unter Gesamtschaft wird die Gesamtheit aus Halsteil, Schaftteil und Fortsatz verstanden. Unter Gesamtlänge wird somit die Länge des Gesamtschafts verstanden.

Nach der Erstimplantation einer Gelenkendoprothese wird nach gewisser Zeit häufig ein Austausch erforderlich, insbesondere wenn die Gelenkendoprothese defekt oder verschlissen ist. Ein hierbei auftretendes Problem ist, dass der aufnehmende Knochen an seiner Oberfläche häufig beschädigt ist.

Ein Knochen zur Aufnahme des Revisionsschafts ist meist von röhrenartiger Gestalt. Sein Endbereich wird als Epimetaphyse bezeichnet (eine Zusammenziehung von Epiphyse und Metaphyse) und der zentrale mittlere (häufig annähernd zylinderförmige) Bereich wird als Diaphyse bezeichnet. Vorliegend ist die Epimetaphyse das Ende des Knochens, an dem der Revisionsschaft mit seinem Halsteil angeordnet ist. Der Schaftteil erstreckt sich von der Epimetaphyse in die Diaphyse. Der an die Diaphyse anschließende Bereich am gegenüberliegenden Ende wird vorliegend als distale Epimetaphyse bezeichnet. "Distal" ist ein Fachbegriff der Anatomie und bezeichnet das Gegenteil von "proximal".

Kern der Erfindung ist der Gedanke, mit dem Fortsatz eine Verlängerung für den Schaft zu schaffen, der über die Diaphyse hinausgehend sich in die jenseitige Epimetaphyse (distale Epimetaphyse) erstreckt, um dort verankert zu werden. Es wird so zum einen ein entfernt liegender Verankerungspunkt genutzt, was aufgrund Hebelwirkung günstige Kraftverhältnisse ermöglicht. Zum anderen wird so ein bisher nicht zur Verankerung genutzter Knochenbereich genutzt, der somit nicht durch Extraktion vorgeschädigt ist. In der Kombination ergibt sich so mit verblüffend einfachen Mitteln eine erheblich verbesserte und sicherere Verankerung, auch bei schwierigen Revisionsfällen.

Die Nutzung der distalen Epimetaphyse für die Verankerung ist bisher nicht Erwägung gezogen worden. Denn dort weitet sich - bezogen auf den engen Querschnitt in der Diaphyse - der Markraum auf. In der Diaphyse sitzt der Prothesenschaft straff durch engen Kontakt mit der Knochenwandung. In der distalen Epimetaphyse weitet sich aber der Markraum auf, so dass dort der Kontakt fehlt und sich keine ausreichende Verankerung ergibt. Der durch die Diaphyse geführte Schaft kann dort keinen Halt finden. So gab es bisher keine Möglichkeit, bei einem Revisionsschaft einen ggf. ungenügenden Halt (zum Beispiel verursacht durch eine zu kurze Befestigungsstrecke in der Diaphyse) zu verbessern.

Die Erfindung hat erkannt, dass mit einer Kombination aus in die distale Epimetaphyse reichendem Fortsatz und dessen Befestigung mittels Knochenzements (oder eines anderen Adhäsionsmittels) eine stabile und sichernde Verankerung erreicht werden kann. Die Erfindung kombiniert auf originelle Weise einen an sich zur zementfreien Implantation vorgesehenen Schaft mit einem in die distale Epimetaphyse reichenden Fortsatz, der zur zementierten Befestigung ausgebildet ist. Damit kombiniert sie die Vorteile der zementlosen Befestigung des Schafts an sich, was gerade für einen Revisionsschaft wichtig ist, mit einer zementierten Befestigung in der distalen Epimetaphyse. Damit löst sie das bisher nicht gelöste Problem einer verbesserten Verankerung des Schafts zum Schutz vor Lockerung. Der erfindungsgemäße Schaft kann somit auch bei schwierigen Fällen, bei denen bisher mangels ausreichender Befestigungsstrecke in der Diaphyse kein ausreichender Halt erzielt werden konnte, eine stabile und dauerhafte Verankerung erreichen. Die Haltbarkeit der Prothese steigt damit deutlich, und das für den Patienten wichtige Risiko von Komplikationen wird deutlich verringert.

Mit Vorteil ist der Fortsatz unitär ausgebildet. Hierunter wird verstanden, dass der Fortsatz über seinen gesamten Querschnitt hinweg gesehen einstückig ist. Damit werden Nachteile vermieden, die aus einer gebauten Struktur (bestehend aus Kern und Mantel) resultieren.

Bezogen auf die Gesamtlänge des Schafts liegt der Übergang zwischen Schaftbereich und Fortsatz vorzugsweise im Bereich von 60-75 % der Gesamtlänge des Schafts. Es hat sich gezeigt, dass mit dieser Dimensionierung in der Diaphyse überwiegend das Schaftteil angeordnet ist, und der Fortsatz nicht (oder nur mit einem kleinen Anteil). Dies ist günstig für die Fixierung, da der Schaftteil für einen zementlosen Sitz ausgebildet ist.

Zweckmäßigerweise bildet der Schaftteil mit dem Fortsatz einen Knickschaft. Das bedeutet, dass der Schaftteil an sich und der Fortsatz mit ihren jeweiligen Mittelachsen nicht parallel sind, sondern diese einen (kleinen) Winkel bilden, den Knickwinkel. Bevorzugte Werte für den Knickwinkel betragen 1° bis 5°. Der Begriff des "Knick" bezieht sich vorliegend auf den Versatz der Mittelachsen; er umfasst sowohl einen scharfen, eng lokalisierten Winkelübergang wie auch einen weichen, sich über eine beträchtliche Strecke erstreckenden bogenartigen Verlauf. Es kann hiermit nicht nur ein leichteres Einführen des Fortsatzes mit dem Schaftteil erreicht werden, sondern der Fortsatz wird so günstig in dem Markraum der distalen Epimetaphyse für eine Befestigung mit einem Adhäsionsmittel (Zement) positioniert. Hierbei kann insbesondere bei einem scharfen Knick die eigentliche Knickstelle genau am Übergang zwischen Schaftteil und Fortsatz liegen. Unbedingt erforderlich ist dies aber nicht. Die Knickstelle kann auch abweichend vom Übergang positioniert sein, vorzugsweise in der Nähe. Bewährt hat sich eine Ausführung des Knicks als generell bogenförmige Gestaltung (ohne eine scharf begrenzte Knickstelle). Der Fortsatz selbst ist dann im distalen Bereich vorzugsweise im Wesentlichen gerade.

Mit Vorteil ist der Fortsatz in dem Bereich seiner Spitze verjüngend ausgeführt. Es wird dadurch das Einführen erleichtert, insbesondere durch die verhältnismäßig enge Diaphyse hindurch.

Vorzugsweise weist der Fortsatz einen unrunden Querschnitt auf, der rotationshemmend geformt ist. Es wird so ein Schutz gegen ein unerwünschtes Verdrehen im Zement erreicht. Die Verankerung mittels Adhäsionsmittel (Zement) erlaubt hier eine freiere Gestaltung des Querschnitts als es bei einer zementfreien Formgebung, auf Einwachsen von Knochenmaterial angewiesenen Form möglich wäre. Vorteilhafte Ausführungsformen für den Querschnitt sind beispielsweise dreiecksförmig oder kreuzförmig, zweckmäßigerweise mit jeweils verrundeten Ecken um ein Einschneiden und Spannungsspitzen zu vermeiden.

Der Fortsatz ist vorzugsweise abhängig von den Abmessungen des Schaftteils dimensioniert. Bewährt hat sich, wenn der Fortsatz so lang ist, dass die Gesamtlänge des Schafts mindestens das 22-fache des Durchmessers beträgt. Damit kann eine günstige, an die anatomischen Anforderungen angepasste Gestaltung erreicht werden.

Häufig wird der Schaftteil einteilig mit dem Fortsatz ausgeführt sein. Das bietet den Vorzug einfacher Anwendbarkeit und günstiger Herstellung. Es kann aber auch vorgesehen sein, dass der Fortsatz gesondert ausgeführt ist. Das bietet den Vorzug, den Fortsatz unabhängig einsetzen zu können. Insbesondere bei anatomisch schwierigen Verhältnissen, wie gekrümmter Diaphyse, bietet dies eine verbesserte Handhabung. Es kann aber auch gemäß einer besonders bevorzugten Ausführungsform vorgesehen sein, eine Kopplungsstelle vorzusehen, die ein Einführen des Fortsatzes von der gegenüberliegenden Seite ermöglicht. Vorzugsweise ist die Kopplungsstelle drehfest im gekoppelten Zustand. Es kann so der Fortsatz zuerst von der anderen Seite (im Fall der Femurkomponente einer Hüftprothese von der tibialen Seite des Femurs) eingebracht und mittels des Adhäsionsmittels (Zement) fixiert werden. Anschließend wird dann wie üblich von der proximalen Seite der Prothesenschaft eingeführt und mit dem Fortsatz verbunden. Damit können auch ansonsten nur schwer beherrschbare Zugangsprobleme gelöst werden.

Vorzugsweise ist ein Zentrierelement für den Fortsatz vorgesehen zur Anordnung in der distalen Epimetaphyse. Damit wird eine Ausrichtung des Fortsatzes vor dem Zementieren erleichtert.

Der Fortsatz ist zweckmäßigerweise so beschaffen, dass er ein Elastizitätsmodul von 100 bis 250 GPa aufweist, vorzugsweise von 180 bis 230 GPa. Es lässt sich so eine gute Anpassung an die Steifigkeit des Knochens und der zementierten Verankerung erreichen. Ein bevorzugtes Material für den Fortsatz bzw. für den gesamten Revisionsprothesenschaft ist Kobalt-Chrom-Molybdän (CoCrMo).

Zweckmäßigerweise ist der Revisionsprothesenschaft modular ausgeführt, insbesondere mit einem austauschbaren Halsteil. Dies ermöglicht durch Verwendung unterschiedlicher Halsteile eine optimale Anpassung an die jeweiligen anatomischen Verhältnisse.

Die Erfindung erstreckt sich ferner auf ein Verfahren zur Implantation des genannten Revisionsprothesenschafts. Bei dem Verfahren zum Implantieren eines Revisonsprothesenschafts einer Revisions-Gelenkendoprothese zur Verankerung in einem epimetaphysären Bereich eines langgestreckten Knochens, insbesondere Femur, mit einer Epimetaphyse, einer Diaphyse und einer distalen Epimetaphyse, wobei der Revisions-Prothesenschaft einen Halsteil am oberen Ende zur Aufnahme eines Gelenkstücks und einen sich an den Halsteil anschließenden Schaftteil aufweist, dessen Oberfläche zur adhäsionsmittel-freien (zementfreien) Befestigung in der Epimetaphyse ausgebildet ist und dessen Länge so bemessen ist, dass sie in die Diaphyse des Knochens reicht, und an dem fernen Ende des Schaftteils ein distaler epimetaphysärer Fortsatz vorgesehen ist, dessen Länge so bemessen ist, dass seine Spitze in die distale Epimetaphyse des Knochens reicht, wobei der Fortsatz zur zementierten Befestigung in der distalen Epimetaphyse ausgebildet ist, sind erfindungsgemäß die Schritte vorgesehen:
Einsetzen des Schafteils mit dem Halsteil von proximal in den epimetaphysären Bereich des langgestreckten Knochens, Schaffen eines Aufnahmebetts aus Adhäsionsmaterial in der distalen Epimetaphyse; Einsetzen des Fortsatzes in die distale Epimetaphyse, wobei der Fortsatz eine Verlängerung des Schaftteils über die Diaphyse hinaus bis in die distale Epimetaphyse bildet.

Nachfolgend werden anhand der Zeichnung vorteilhafte Ausführungsformen der Erfindung beispielhaft erläutert. Es zeigen:
- Fig. 1: eine Ansicht eines Revisionsprothesenschafts in der Projektion auf die LM-Ebene;
- Fig. 2: eine Querschnitt-Darstellung eines Schaftteils des Revisionsprothesenschafts;
- Fig. 3a-c: alternative Querschnitte des Fortsatzes;
- Fig. 4: eine Kopplung zwischen Schaftteil und Fortsatz;
- Fig. 5: eine alternative Ausführungsform für einen Revisionsprothesenschaft;
- Fig. 6: ein Zentrierelement am Fortsatz;
- Fig. 7: einen Prothesensatz mit alternativen Halsteilen;
- Fig. 8: eine Abfolge beim Einsetzen des Revisionsprothesenschafts; und
- Fig. 9: eine alternative Abfolge beim Einsetzen des Revisionsprothesenschafts.

Bei der in den Figuren dargestellten Ausführungsform handelt es sich um eine Femurkomponente einer Hüftgelenkendoprothese, die außer der Femurkomponente ferner eine Acetabulumkomponente (nicht dargestellt) umfasst.

Die Femurkomponente umfasst einen Revisionsschaft 1 mit einem Halsteil 11 im oberen proximalen Bereich und einem Schaftteil 12 im unteren distalen Bereich. Sie wird eingesetzt in einen langgestreckten Röhrenknochen, bei dem es sich vorliegend um einen Femur 9 handelt. Dieser weist an seinem oberen, dem Acetabulum (nicht dargestellt) zugewandten Ende eine Epimetaphyse 91 auf, in seinem mittleren Bereich eine Diaphyse 92 und in seinem unteren, zur Tibia (nicht dargestellt) hin gewandten Bereich eine weitere Epimetaphyse auf, die vorliegend als distale Epimetaphyse 93 bezeichnet ist.

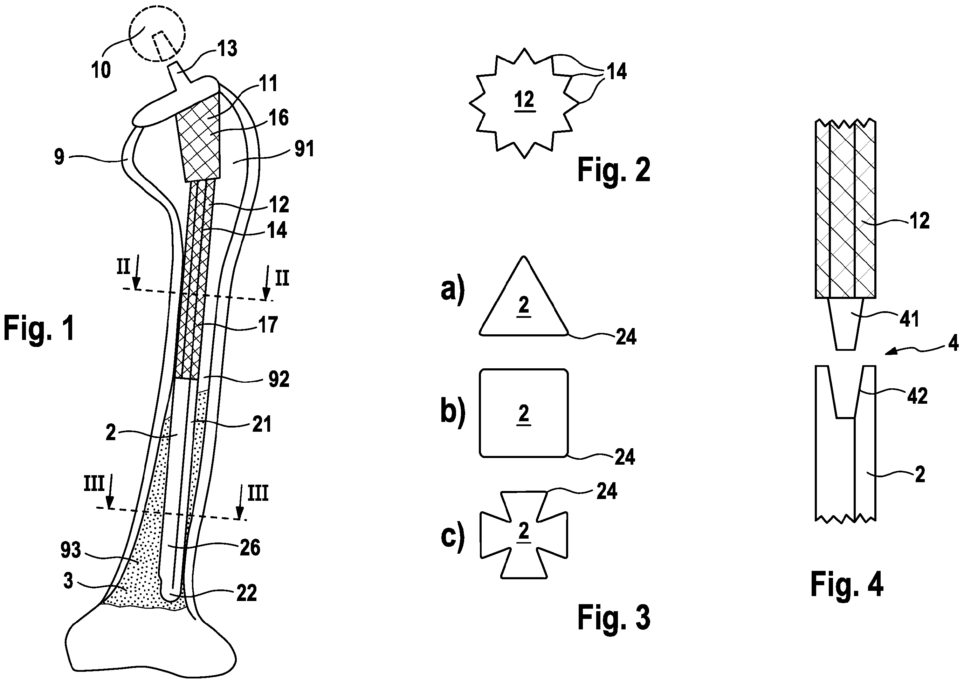

Der Halsteil 11 ist von oben in die Epimetaphyse 91 des Femurs 9 eingesetzt. Der Halsteil weist an seinem oberen Ende einen schräg nach oben weisenden Haltekonus 13 auf. Auf diesem wird ein kugelförmiger Gelenkkopf 10 angeordnet, der zu diesem Zweck eine Konusbohrung zum Aufsetzen auf den Haltekonus 13 aufweist. Der Halsteil 11 ist verbreitert in seiner Ausdehnung zwischen seiner medialen Begrenzung (in Figur 1 links dargestellt) und seiner lateralen Begrenzung (in Figur 1 rechts dargestellt). Der Halsteil 11 ist so geformt, dass er die Epimetaphyse 91 des Femurs 9 weitgehend ausfüllt und mit seinen lateralen Seiten lasttragend an der Innenseite der Knochenwandung anliegt.

Der Schaftteil 12 ist an dem dem Gelenkkopf 10 gegenüberliegenden Ende an dem Halsteil 11 angeordnet. Er schließt sich mit fließenden Übergängen an das Halsteil 11 an. Der Schaftteil 12 ist von generell lang gestreckter Gestalt und ist im Wesentlichen stabförmig ausgebildet. In seinem mittleren und unteren Bereich weist der Schaftteil 12 einen im Wesentlichen runden Querschnitt mit einer Mehrzahl von Längsrippen 14 auf, wie in Fig. 2 dargestellt. Der Querschnitt ist so bemessen, dass er einen Markkanal in der Diaphyse 92 des Femurs 9 ausfüllt.

Der Revisionsschaft 1 ist aus einem biokompatiblen Material hergestellt. Bei der dargestellten Ausführungsform wird als Material Kobalt-Chrom-Molybdän (CoCrMo) verwendet. Der Halsteil 11 und der Schaftteil 12 sind mit einer Beschichtung 16, 17 versehen, die das Einwachsen von Knochenmaterial begünstigt (hierbei kann es sich beispielsweise um Titan oder um eine Beschichtung mit Titan oder Hydroxilapatit handeln). Auf diese Weise wird durch das Einwachsen von Knochenmaterial eine zementfreie, aber dennoch feste Verankerung des Revisionsschafts 1 erreicht.

An dem unteren, fernen Ende des Schaftteils 12 ist ein Fortsatz 2 angeordnet. Der Fortsatz 2 ist ebenfalls von stabartiger Gestalt. Er ist massiv ausgeführt und unitär aus einem Material hergestellt. Bei dem Material handelt es sich vorzugsweise ebenfalls um Kobalt-Chrom-Molybdän (CoCrMo). Der Fortsatz 2 ist so gestaltet, dass seine Oberfläche 26 zur zementierten Implantation ausgebildet ist. Das obere Ende 21 des Fortsatzes 2 ist ferner so gestaltet, dass es die Außenkontur des unteren Endes des Schaftteils 12 ohne Versatz fortsetzt. Das untere Ende des Fortsatzes 2 ist atraumatisch gestaltet mit einer verrundeten Spitze 22. Die Spitze 22 weist einen geringeren Querschnitt auf als das obere Ende 21, sodass sich der Fortsatz 2 in dem Bereich seiner Spitze 22 nach unten hin verjüngt.

Der Fortsatz 2 weist vorzugsweise keine runde Gestaltung auf, sondern vielmehr einen unrunden Querschnitt. Damit kann der Fortsatz 2 zur Rotationssicherung beitragen. Während im oberen Bereich des Fortsatzes der Querschnitt noch angepasst an denjenigen des unteren Endes des Schaftteils 12 ist, verändert er sich nach unten hin und ist im mittleren bzw. unteren Bereich deutlich unrund gestaltet, beispielsweise dreiecksförmig oder kleeblattförmig. Beispiele für solche Querschnitte sind in Fig. 3 dargestellt. Zur Traumavermeidung sind die Rippen 24 bildenden Ecken hierbei vorzugsweise verrundet ausgeführt, so dass keine Gefahr von Einschnitten bzw. Einkerbung besteht.

Zur Fixierung des Fortsatzes 2 ist ein Zementbett 3 vorgesehen. Es ist in der distalen Epimetaphyse 93 angeordnet und reicht bis in die Diaphyse 92. Das Zementbett 3 umhüllt den Fortsatz 2 und befestigt diesen damit entlang einer Strecke, die weit über die Diaphyse 92 hinausragt bis tief in die distale Epimetaphyse 93. Damit ist der Fortsatz 2 nicht nur in dem engen Querschnitt des Markkanals in der Diaphyse 92, sondern dank des Zementbetts 3 auch in dem verhältnismäßig weiten Querschnitt der distalen Epimetaphyse 93 sicher und stabil befestigt. Die insgesamt wirksame Befestigungsstrecke vergrößert sich dadurch erfindungsgemäß beträchtlich, was zu der verbesserten Befestigungsqualität führt.

Die unrunde Form des Querschnitts (siehe Fig. 3) im mittleren und unteren Bereich des Fortsatzes 2 bewirkt in Kombination mit dem Zementbett 3 zusätzlich noch eine effektive Sicherung gegen unerwünschte Rotation.

Eine Detailvergrößerung eines Übergangs mit einer Kopplung 4 zwischen dem Schaftteil 12 und dem Fortsatz 2 ist in Fig. 4 dargestellt. Die Kopplung 4 ist als Konusverbindung ausgeführt mit einem an dem oberen Ende des Fortsatzes 2 angeordneten Außenkonus 42, der in einen komplementär geformten Innenkonus 41 am unteren Ende des Schaftteils 12 greift (es sei angemerkt, dass die Anordnung von Außen- und Innenkonus auch umgekehrt sein kann). Mit dieser Konusverbindung wird eine kraftschlüssige und sichere Kopplung des Fortsatzes 2 an den Schaftteil 12 des Revisionsschafts 1 geschaffen. Hierbei ergibt sich im eingesetzten Zustand aufgrund der Klemmwirkung der Konusverbindung ferner eine drehfeste Verbindung zwischen Fortsatz 2 und Schaftteil 12. Die Kopplung 4 definiert hierbei den Übergang zwischen Schaftteil 12 und Fortsatz 2. Im Fall einer einteiligen Ausführung ohne Kopplung ist der Übergang definiert von der Oberflächengestaltung für zementfreie Implantation am Schaftteil 12 zur zementierten Implantation am Fortsatz 2.

Mit dieser durch die Konusverbindung geschaffenen Kopplung 4 kann der Fortsatz 2 zum einen bereits vormontiert sein, so dass er zusammen mit dem Schaftteil 12 von oben in den Femur 9 eingeführt wird. Es kann alternativ aber auch vorgesehen sein, dass der Fortsatz 2 unabhängig von dem Schaftteil 12 montiert wird. Hierbei kann die unabhängige Montage von oben oder, was in bestimmten Fällen Vorzüge für sich buchen kann, auch von der anderen Seite, also von unten erfolgen. Letzteres bietet den Vorteil, dass hierbei der Fortsatz 2 direkt in die distale Epimetaphyse 93 eingesetzt werden kann, ohne vorher durch die Diaphyse 92 geschoben zu werden. Dies ermöglicht den Einsatz stärker dimensionierter Fortsätze. Deren Weite ist nicht mehr durch den verhältnismäßig engen Durchgangsquerschnitt der Diaphyse 92 beschränkt. Das kann optional dazu verwendet werden, einen Fortsatz 2 mit größerem Durchmesser zu verwenden als den eigentlichen Schaft 12, um so noch mehr Stabilität zu erhalten.

Die Befestigung des Fortsatzes 2 erfolgt, wie bereits ausgeführt, im Wesentlichen durch das Zementbett 3. Dies gilt besonders für den Bereich der distalen Epimetaphyse 93. Um hierbei eine stabile initiale Positionierung an der gewünschten Stelle sicherzustellen, kann ein Zentrierstern 5 vorgesehen sein. Dieser ist dazu ausgebildet, auf das untere, freie Ende mit der Spitze 22 des Fortsatzes 2 aufgeschoben zu werden. Der Zentrierstern 4 ist innenseitig auf dem Fortsatz 2 gehaltert und stützt sich außen über mehrere, umlaufend verteilte Auflagepunkte 50 an der Knochenwandung des Femurs 9 im Bereich der distalen Epimetaphyse 93 ab. Die Verwendung des Zentriersterns 5 ist insbesondere dann von Vorteil, wenn der Fortsatz 2 vorab von unten direkt in die distale Epimetaphyse 93 eingesetzt wird. Jedoch ist seine Anwendung nicht auf diesen Fall beschränkt.

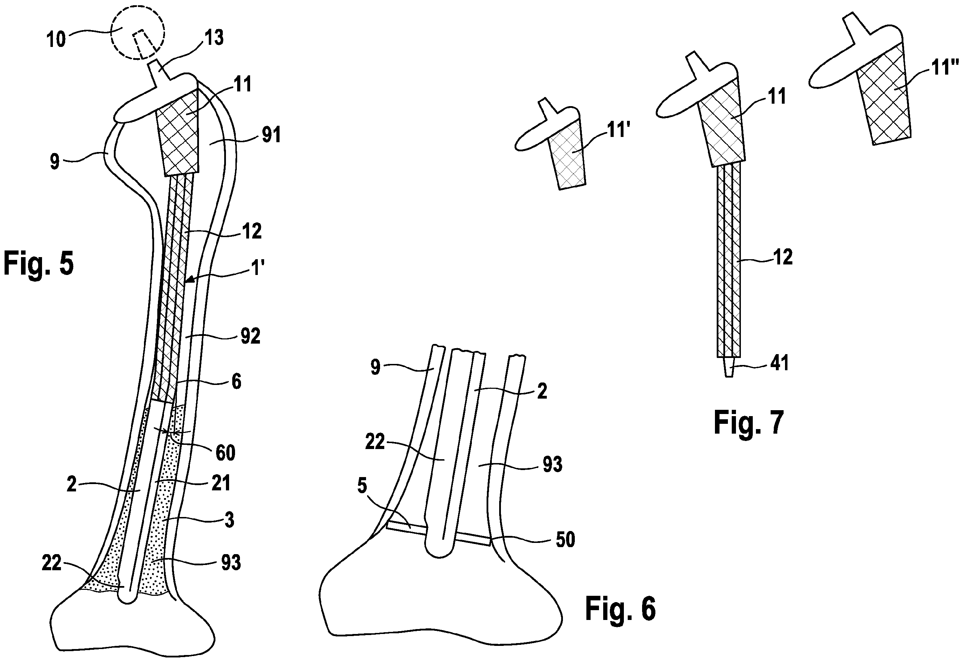

Alternativ kann der Revisionsprothesenschaft als ein Knickschaft 1' ausgeführt sein. Er weist eine Knickstelle 6 auf, so dass der Fortsatz 2 nicht koaxial mit dem Schaftteil 12 ist, sondern gegenüber diesem einen Knickwinkel 60 aufweist. Der Knickwinkel in dem dargestellten Ausführungsbeispiel soll 3° betragen (in Fig. 5 zur Verdeutlichung übergroß dargestellt). In dem dargestellten Ausführungsbeispiel ist der Schaft 1 einstückig mit dem Fortsatz 2 ausgeführt. Es sei angemerkt, dass dies auch bei der geraden, nicht geknickten Variante so vorgesehen sein kann.

Die Erfindung kann als ein Prothesensystem ausgeführt sein. Hierbei können unterschiedliche Halsteile 11, 11', 11" mit demselben oder verschiedenen Schafteil 12 verknüpft werden zur Anpassung an verschiedene anatomische Gegebenheiten.

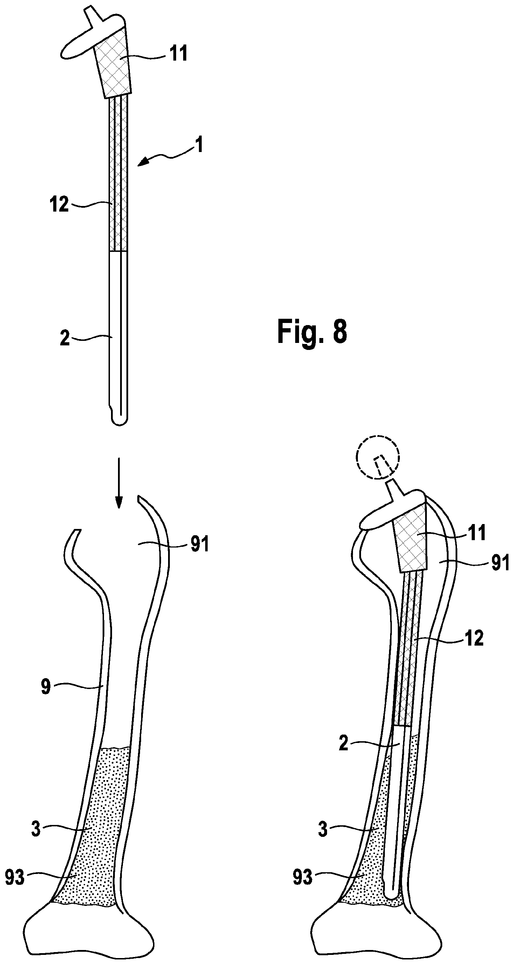

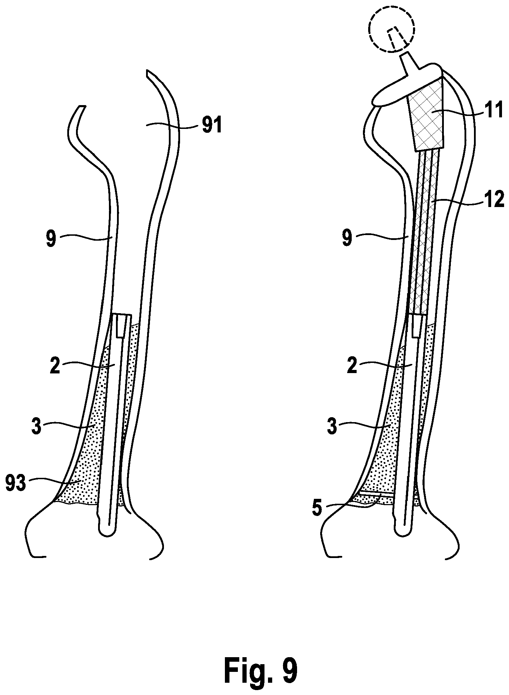

Das Verfahren zum Einsetzen des erfindungsgemäßen Revisionsschafts 1 ist anhand zweier verschiedener Beispiele in den Figuren 8 bzw. 9 dargestellt. Figur 8 zeigt den Fall, dass der Revisionsschaft als eine Einheit (sei es einstückig ausgeführt oder mit bereits in den Schaftteil 12 eingesetztem Fortsatz 2) von oben durch die Epimetaphyse 91 eingesetzt wird. Hierbei wird der Fortsatz 2 durch die Diaphyse 92 hindurchgeschoben, bis er mit seinem unteren Bereich mit der Spitze 22 die distale Epimetaphyse 93 erreicht hat. Hierbei ist vorzugsweise bereits vor dem Einsetzen ein Zementbett 3 in der distalen Epimetaphyse 93 geschaffen worden. Dies kann beispielsweise mittels einer Zementspritze (nicht dargestellt) erfolgen. Es erfolgt dann mit dem Einschieben des Revisionsprothesenschafts 1 mit angesetztem Fortsatz 2 unmittelbar eine Verankerung des Fortsatzes 2 in dem Zementbett 3. Damit ist der Revisionsprothesenschaft 1 sicher gehaltert, und zwar auch in der distalen Epimetaphyse 93 mit ihrem weiten Querschnitt.

Alternativ kann aber auch vorgesehen sein, dass der Fortsatz 2 unabhängig eingesetzt wird, und zwar vorzugsweise von der Gegenseite aus. Dies ist in Fig. 9 dargestellt. Hierzu wird der Fortsatz 2 von unten direkt in die distale Epimetaphyse 93 eingeschoben, und zwar so weit, bis der Fortsatz 2 mit seinem oberen Ende und dem daran angeordneten Verbindungsteil (Innenkonus 42) in die Diaphyse 92 eingeschoben ist. Der mittlere und untere Bereich mit der Spitze 22 des Fortsatzes wird dann mittels des Zementbetts 3 in der distalen Epimetaphyse 93 befestigt. Hierbei kann in besonders zweckmäßiger Weise der besagte Zentrierstern 5 verwendet werden, um eine korrekte Positionierung des Fortsatzes 2 in der distalen Epimetaphyse 93 zu gewährleisten. In einem nächsten Schritt wird der Halsteil 11 mit dem Schaftteil 12 von oben durch die Epimetaphyse 91 eingesetzt, wobei der Schaftteil 12 in die Diaphyse 92 vorgeschoben wird und dort mit seinem komplementären Außenkonus 41 verbunden wird mit dem Innenkonus 42 am Fortsatz 2.

Das letztgenannte Verfahren bietet sich insbesondere dann an, wenn Halsteile 11 mit unterschiedlichen Größen verwendet werden sollen. Hierbei kann unabhängig von der Implantation des Fortsatzes 2 je nach anatomischen Anforderungen die jeweilige Größe ausgewählt werden. Beispiele für unterschiedlich große Halsteile 11, 11', 11" eines modularen Prothesensatzes sind in Fig. 7 dargestellt. Sie werden auf das Schaftteil 12 aufgesetzt und bilden so eine Einheit, die gemeinsam eingesetzt wird, wobei mittels der Kopplung 4 eine sichere Verbindung mit dem Fortsatz 2 erreicht wird.

## Patentansprüche

1. Revisionsprothesenschaft einer Revisions-Gelenkendoprothese zur Verankerung in einem epimetaphysären Bereich eines langgestreckten Knochens (9), insbesondere Femur, der eine Epimetaphyse (91), eine Diaphyse (92) und eine distale Epimetaphyse (93) umfasst, wobei der Revisionsprothesenschaft (1) einen Halsteil (11) am oberen Ende zur Aufnahme eines Gelenkstücks (10) und ein sich an den Halsteil (11) anschließenden Schaftteil (12) aufweist, dessen Oberfläche zur adhäsionsmittel-freien Befestigung in der Epimetaphyse (91) und Diaphyse (92) ausgebildet ist und dessen Länge so bemessen ist, um in die Diaphyse (92) des Knochens zu reichen,
**dadurch gekennzeichnet, dass**
an dem fernen Ende des Schaftteils (12) ein distaler epimetaphysärer Fortsatz (2) für den Schaft vorgesehen ist, dessen Länge so bemessen ist, dass seine Spitze in die distale Epimetaphyse (93) des Knochens reicht, wobei der Fortsatz (2) zur Befestigung mit einem Adhäsionsmittel (3), insbesondere Knochenzement, in der distalen Epimetaphyse (93) ausgebildet ist.

2. Revisionsprothesenschaft nach Anspruch 1, **dadurch gekennzeichnet, dass** der Fortsatz (2) unitär ausgebildet ist.

3. Revisionsprothesenschaft nach einem der vorangehenden Ansprüche, **dadurch gekennzeichnet, dass** ein Übergang zwischen Schaftteil (12) und Fortsatz (2) im Bereich von 40-60 % der Gesamtlänge des Schafts (1) liegt.

4. Revisionsprothesenschaft nach einem der vorangehenden Ansprüche, **dadurch gekennzeichnet, dass** der Schaftteil (12) mit dem Fortsatz (2) einen Knickschaft (1') bildet, vorzugsweise mit einem Knickwinkel von 1° bis 5°.

5. Revisionsprothesenschaft nach Anspruch 3 und 4, **dadurch gekennzeichnet, dass** eine Knickstelle im Bereich des Übergangs, aber versetzt zum Übergang liegt.

6. Revisionsprothesenschaft nach einem der vorangehenden Ansprüche, **dadurch gekennzeichnet, dass** der Fortsatz (2) in dem Bereich seiner Spitze (22) verjüngend ausgeführt ist.

7. Revisionsprothesenschaft nach einem der vorangehenden Ansprüche, **dadurch gekennzeichnet, dass** der Fortsatz (2) in seinem distalen Bereich im Wesentlichen gerade ist.

8. Revisionsprothesenschaft nach einem der vorangehenden Ansprüche, **dadurch gekennzeichnet, dass** der Fortsatz (2) einen unrunden Querschnitt aufweist, der rotationshemmend geformt ist, vorzugsweise dreiecksförmig oder kreuzförmig mit jeweils verrundeten Ecken.

9. Revisionsprothesenschaft nach einem der vorangehenden Ansprüche, **dadurch gekennzeichnet, dass** der Fortsatz (2) so lang ist, dass die Gesamtlänge des Schafts (1) mindestens das 22-fache des Durchmessers beträgt.

10. Revisionsprothesenschaft nach einem der vorangehenden Ansprüche, **dadurch gekennzeichnet, dass** der Schaftteil (12) einteilig mit dem Fortsatz (2) ausgeführt ist.

11. Revisionsprothesenschaft nach einem der Ansprüche 1 bis 9, **dadurch gekennzeichnet, dass** der Fortsatz (2) gesondert ausgeführt ist und eine Kopplungsstelle (4) zur drehfesten Verbindung des Fortsatzes (2) mit dem Schaftteil (12) vorgesehen ist.

12. Revisionsprothesenschaft nach einem der vorangehenden Ansprüche, **dadurch gekennzeichnet, dass** ein Zentrierelement (5) am Fortsatz (2) vorgesehen ist.

13. Revisionsprothesenschaft nach einem der vorangehenden Ansprüche, **dadurch gekennzeichnet, dass** der Fortsatz (2) ein Elastizitätsmodul von 100 bis 250 GPa, vorzugsweise von 180 bis 230 GPa aufweist, insbesondere mit CoCrMo als Material.

14. Revisionsprothesenschaft nach einem der vorangehenden Ansprüche, **dadurch gekennzeichnet, dass** der Schaft (1) modular mit einem austauschbaren Halsteil (11) ausgeführt ist.

## Claims

1. Revision prosthesis shaft of a revision joint endoprosthesis for anchoring in an epimetaphyseal region of an elongate bone (9), in particular femur, which comprises an epimetaphysis (91), a diaphysis (92) and a distal epimetaphysis (93), wherein the revision prosthesis shaft (1) has, at the upper end, a neck part (11) for receiving a joint piece (10) and, adjoining the neck part (11), a shaft part (12) whose surface is designed for adhesive agent-free fastening in the epimetaphysis (91) and diaphysis (92) and whose length is dimensioned such that it reaches into the diaphysis (92) of the bone,
**characterized in that**
a distal epimetaphyseal extension (2) for the shaft is provided at the far end of the shaft part (12), the length of which extension (2) is dimensioned such that its tip reaches into the distal epimetaphysis (93) of the bone, wherein the extension (2) is designed for fastening in the distal epimetaphysis (93) with an adhesive agent (3), in particular bone cement.

2. Revision prosthesis shaft according to Claim 1, **characterized in that** the extension (2) is configured as a single piece.

3. Revision prosthesis shaft according to either of the preceding claims, **characterized in that** a transition between shaft part (12) and extension (2) is in the region of 40-60% of the total length of the shaft (1).

4. Revision prosthesis shaft according to one of the preceding claims, **characterized in that** the shaft part (12) forms, with the extension (2), a kinked shaft (1'), preferably with a kink angle of 1° to 5°.

5. Revision prosthesis shaft according to Claims 3 and 4, **characterized in that** a kink site lies in the region of the transition, but offset in relation to the transition.

6. Revision prosthesis shaft according to one of the preceding claims, **characterized in that** the extension (2) has a tapering configuration in the region of its tip (22).

7. Revision prosthesis shaft according to one of the preceding claims, **characterized in that** the extension (2) is substantially straight in its distal region.

8. Revision prosthesis shaft according to one of the preceding claims, **characterized in that** the extension (2) has a non-round cross section which is shaped to inhibit rotation, preferably triangular or cross-shaped, in each case with rounded corners.

9. Revision prosthesis shaft according to one of the preceding claims, **characterized in that** the extension (2) is of such a length that the total length of the shaft (1) is at least 22 times the diameter.

10. Revision prosthesis shaft according to one of the preceding claims, **characterized in that** the shaft part (12) is configured in one piece with the extension (2).

11. Revision prosthesis shaft according to one of Claims 1 to 9, **characterized in that** the extension (2) is configured separately, and a coupling site (4) is provided for rotationally fixed connection of the extension (2) to the shaft part (12).

12. Revision prosthesis shaft according to one of the preceding claims, **characterized in that** a centring element (5) is provided on the extension (2).

13. Revision prosthesis shaft according to one of the preceding claims, **characterized in that** the extension (2) has an elastic modulus of 100 to 250 GPa, preferably of 180 to 230 GPa, in particular with CoCrMo as material.

14. Revision prosthesis shaft according to one of the preceding claims, **characterized in that** the shaft (1) is of a modular configuration with an exchangeable neck part (11).

## Revendications

1. Tige de prothèse de révision pour une endoprothèse articulaire de révision pour l'ancrage dans une région épimétaphysaire d'un os allongé (9), en particulier un fémur, qui comprend une épimétaphyse (91), une diaphyse (92) et une épimétaphyse distale (93), dans laquelle la tige de prothèse de révision (1) présente une partie de col (11) à l'extrémité supérieure pour le logement d'une pièce d'articulation (10) et une partie de tige (12) se raccordant à la partie de col (11), dont la surface est réalisée en vue de la fixation sans agent adhésif dans l'épimétaphyse (91) et la diaphyse (92) et dont la longueur est dimensionnée de façon à pénétrer dans la diaphyse (92) de l'os, **caractérisée en ce qu'**il est prévu à l'extrémité éloignée de la partie de tige (12) un prolongement épimétaphysaire distal (2) pour la tige, dont la longueur est dimensionnée de telle manière que sa pointe pénètre dans l'épimétaphyse distale (93) de l'os, dans laquelle le prolongement (2) est réalisé en vue de la fixation avec un agent adhésif (3), notamment un ciment osseux, dans l'épimétaphyse distale (93).

2. Tige de prothèse de révision selon la revendication 1, **caractérisée en ce que** le prolongement (2) est réalisé de façon unitaire.

3. Tige de prothèse de révision selon l'une quelconque des revendications précédentes, **caractérisée en ce qu'**une transition entre la partie de tige (12) et le prolongement (2) se situe dans la région de 40 - 60 % de la longueur totale de la tige (1).

4. Tige de prothèse de révision selon l'une quelconque des revendications précédentes, **caractérisée en ce que** la partie de tige (12) forme avec le prolongement (2) une tige coudée (1'), de préférence avec un angle de coude de 1° à 5°.

5. Tige de prothèse de révision selon une revendication 3 ou 4, **caractérisée en ce qu'**un coude se situe dans la région de la transition, mais décalé par rapport à la transition.

6. Tige de prothèse de révision selon l'une quelconque des revendications précédentes, **caractérisée en ce que** le prolongement (2) est réalisé avec un amincissement dans la région de sa pointe (22).

7. Tige de prothèse de révision selon l'une quelconque des revendications précédentes, **caractérisée en ce que** le prolongement (2) est essentiellement rectiligne dans sa région distale.

8. Tige de prothèse de révision selon l'une quelconque des revendications précédentes, **caractérisée en ce que** le prolongement (2) présente une section transversale non ronde, qui est formée de façon à empêcher la rotation, de préférence en forme de triangle ou en forme de croix avec des angles chaque fois arrondis.

9. Tige de prothèse de révision selon l'une quelconque des revendications précédentes, **caractérisée en ce que** le prolongement (2) est d'une longueur telle que la longueur totale de la tige (1) vaut moins 22 fois le diamètre.

10. Tige de prothèse de révision selon l'une quelconque des revendications précédentes, **caractérisée en ce que** la partie de tige (12) est réalisée d'un seul tenant avec le prolongement (2).

11. Tige de prothèse de révision selon l'une quelconque des revendications 1 à 9, **caractérisée en ce que** le prolongement (2) est réalisé séparément et il est prévu un point de couplage (4) pour la liaison calée en rotation du prolongement (2) avec la partie de tige (12).

12. Tige de prothèse de révision selon l'une quelconque des revendications précédentes, **caractérisée en ce qu'**il est prévu un élément de centrage (5) sur le prolongement (2).

13. Tige de prothèse de révision selon l'une quelconque des revendications précédentes, **caractérisée en ce que** le prolongement (2) présente un module d'élasticité de 100 à 250 GPa, de préférence de 180 à 230 GPa, en particulier avec un matériau constitué de CoCrMo.

14. Tige de prothèse de révision selon l'une quelconque des revendications précédentes, **caractérisée en ce que** la tige (1) est réalisée de façon modulaire avec une partie de col échangeable (11).
